# EUROPEAN PATENT SPECIFICATION

(11) **EP 2 424 612 B1**
(45) Date of publication and mention of the grant of the patent: **07.08.2013**
(21) Application number: 10716217.4
(22) Date of filing: 23.04.2010
(51) Int. Cl.: A61M 39/10, A61M 5/34

(54) **LUER SLIP CONNECTOR WITH ROUGHENED TIP**
LUER-SLIP-STECKER MIT AUFGERAUTER SPITZE
RACCORD A GLISSEMENT LUER A EMBOUT RUGUEUX

(30) Priority: 01.05.2009 US 434251
(43) Date of publication of application: 07.03.2012
(73) Proprietor: Becton, Dickinson and Company, Franklin Lakes, NJ 07417 (US)
(72) Inventor: KOMMIREDDY, Dinesh, S., Tarrytown NY 10591 (US); LIN, Angela, Wayne NJ 07470 (US); ZHAO, Joyce, Mahwah NJ 07430 (US); PAWLOWSKI, John, Park Ridge NJ 07656 (US)
(74) Representative: von Kreisler Selting Werner
(86) International application number: PCT/US2010/032203
(87) International publication number: WO 2010/126791

(56) References cited:
- EP-A2- 0 813 884
- EP-A2- 1 466 644
- WO-A1-2009/002474
- US-A- 5 312 377
- US-A- 5 851 201
- US-A1- 2009 177 186

## Description

### FIELD OF THE INVENTION

The present invention relates in general to the connector portion of a medical device. More particularly, embodiments of the disclosed invention are directed to the surface finish of a luer slip fitting having a conical tip. Methods of manufacturing a male luer slip fitting device with a roughened surface are also disclosed.

### BACKGROUND OF THE INVENTION

Medical devices having male luer slip fittings, for example, male luer syringes, are often assembled with female tube adapters or tube extensions See in particular document US-A-5,851,201. As described in International Standard ISO 594-1, published by the International Organization for Standardization, such devices include a male conical fitting. Female luer fittings are assembled to the male conical tip by applying axial force to the female luer fitting until the female fitting is sufficiently tight.

Luer slip connections may become loose due to the presence of lubricious materials, such as glycerol monostearate (GMS). GMS is an additive in polypropylene that acts as an antistatic agent. The GMS migrates to the surface over time and causes the coefficient of friction (COF) to drop. GMS is an example of a lubricious material that migrates to the surface, other materials such as a slip agent or other lubricious materials that migrate or are present on the surface of the syringe tip can cause a drop in the COF. Such loose connections could result in leakage of potentially harmful drugs during various medical procedures and result in user dissatisfaction and other complications. Therefore, there exists a need in the art for a luer syringe or fitting that resists or minimizes the drop in COF resulting from lubricious materials without leaking.

### SUMMARY OF THE INVENTION

One or more embodiments of the invention pertain to medical devices comprising a male luer slip fitting connectable to a female luer portion of a second medical device by application of an engagement force to provide a contact surface between the male luer slip fitting and female luer portion and separable by application of a disengagement force that does not require substantial twisting motion. The male luer slip fitting comprises a material incorporating a lubricious agent such that the disengagement force of a dry connection is less than the engagement force when the contact surface does not include a roughened surface, and the male luer slip fitting has contact surface that is roughened such that the disengagement force required to separate the male luer slip fitting from the female luer portion of the second medical device when in a wet connection is greater than the force required to disengage the male luer fitting from the female luer portion of the second medical device when the contact surface of the male luer fitting is not roughened.

Another aspect of the invention pertains to methods of making a medical device comprising manufacturing a male luer fitting with a conical tip having an outside surface that mates with a female luer fitting of a second medical device. The male luer fitting being manufactured from a material containing a lubricious agent such that the force required to disassemble a non-roughened male conical tip from a female luer fitting is less than the force required to assemble the non-roughened male luer fitting from the female luer fitting. The outside surface of the male conical tip is roughened such that upon assembly of the male luer fitting with the female luer fitting, with liquid present between the male conical tip and female luer fitting, the removal force is less than about the assembly force.

### BRIEF DESCRIPTION OF THE DRAWING

So that the manner in which the above recited features of the present invention can be understood in detail, a more particular description of the invention, briefly summarized above, may be had by reference to embodiments.
FIG. 1. shows a syringe with a luer slip and a luer connector:
FIG. 2 shows the hub pull force at various assembly forces for luer fittings made of different materials;
FIG. 3 shows the hub pull force for various materials and surface finishes when assembled at known forces; and
FIG. 4 shows the hub pull force in materials having a lubricious agent when assembled at various forces with various surface finishes.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

As used herein, "luer slip" or "luer fitting" used in conjunction with luer connectors and other luer fittings means a connector that is disassembled primarily by the application of axial force as described in ISO 594-1, as distinguished from luer lock fittings that are disassembled primarily by the application of torque, as described in ISO 594-2.

It has been determined that when a male luer fitting with a conical tip and a female luer fitting are assembled in the presence of a lubricious material under wet conditions, the force required to disassemble the fitting decreases, potentially resulting in leakage. This is especially problematic because plastic formulations often have additives, like glycerol monostearate, designed to reduce friction or the static properties of the material. Without being bound by any particular theory, it is believed that these additives may migrate to the surface of the material resulting in lubricious material being present at the surface. This can cause a decrease in the coefficient of friction of the material resulting in a lower pull force required to disengage a luer connection. It has been found that adjusting the surface finish on at least the male conical tip of a luer connection of a medical device results in an increase in the force required to disengage the fitting.

This is a surprising result as it has long been known that increasing the surface roughness of a luer fitting increases the tendency for leakage during injection. Therefore, it was unexpected to find that increasing the surface roughness could, instead, result in a connection with high pull forces with little or no leakage.

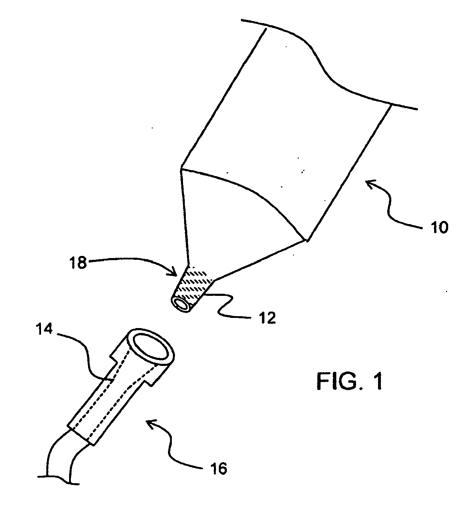

With reference to FIG. 1, one or more embodiments of the invention are directed to medical devices 10 comprising a male luer slip fitting 12. The male luer slip fitting 12 being connectable to a female luer portion 14 of a second medical device 16 by application of an engagement force to provide a contact surface between the male luer slip fitting 12 and female luer portion 14, and separable by application of a disengagement force that does not require substantial twisting motion, meaning that in normal use, the male luer slip fitting 12 and female luer portion 14 do not require more than 90 degrees of relative radial movement. Normally; the male luer slip fitting 12 and female luer portion 14 are assembled by simply applying an axial force in the direction of the longitudinal axis of each fitting by pressing the parts together. It will be appreciated that during normal assembly some small amount of relative twisting between the male luer slip fitting 12 and female luer portion 14 may be utilized by the practitioner to assembly the fittings. The male luer slip fitting 12 comprises a material incorporating a lubricious agent such that the disengagement force of a dry connection is less than the engagement force when the contact surface does not include a roughened surface. The male luer slip fitting 12 has a contact surface that is roughened 18 such that the disengagement force required to separate the male luer slip fitting 12 from the female luer portion 14 of the second medical device 16, when in a wet connection, is greater than the force required to disengage the male luer fitting 12 from the female luer portion 14 of the second medical device 16 when the contact surface of the male luer fitting 12 is not roughened.

Another aspect of the invention pertains to methods of making a medical device. The methods comprise manufacturing a male luer fitting with a conical tip having an outside surface that mates with a female luer fitting of a second medical device. The male luer fitting is manufactured from a material which contains a lubricious agent such that the force required to disassemble a non-roughened male conical tip from a female luer fitting is less than the force required to assemble the non-roughened male luer fitting from the female luer fitting. The outside surface of the male conical tip is roughened such that upon assembly of the male luer fitting with the female luer fitting, with liquid present between the male conical tip and female luer fitting, the removal force is less than about the assembly force.

In other embodiments, the force required to disengage the male luer fitting from the female luer portion increases with increasing engagement force. In specific embodiments, the force required to disengage the male luer fitting from the female luer portion is less than about 80% of the engagement force. In more specific embodiments, the force required to disengage the male luer fitting from the female luer portion is less than about 75%, 70% or 65% of the engagement force.

The surface of the male luer in various embodiments is roughened using a mold to form the tip. The mold has been roughened using a technique selected from Electrodischarge Machining (EDM), vapor honing, cross hatching, polishing and combinations thereof. The external surface of the male luer fitting of some embodiments is roughened to between about 0.3 *µ*m to about 1.2 *µ*m. In specific embodiments, the external surface of the male luer fitting is roughened to between about 0.4 *µ*m to about 0.8 *µ*m. In other specific embodiments. the surface is roughened to have an average roughness of about 0.3 *µ*m or about 0.4 *µ*m or about 0.7 *µ*m or about 1.2 *µ*m. In further specific embodiments, the roughened surface is prepared by electrodischarge machining. The roughened surface of detailed embodiments has a surface with a random patterned finish, i.e., there is not obviously repeatable pattern to the finish. The surface of other detailed embodiments has a repeatable pattern.

In one or more embodiments of the invention, the medical device is made of a material that includes an additive that enhances the lubricious property of the material. The additive of detailed embodiments is glycerol monostearate. In other detailed embodiments, the additive is a slip agent. In still further detailed embodiments, the slip agent is a fatty acid amide.

The invention will be further described with reference to examples.

### Examples A-E

Luer slip syringes made from a resin which lacked glycerol monostearate, or other lubricious agents, were assembled with a needle tip luer fitting. Assembly occurred under controlled axial forces at 3, 6, 8, 10 and 12 lb. The hub pull force required to disassemble the syringe from the luer fitting was measured.

### Examples F-J

Luer slip syringes made from a resin which included a lubricious agent, specifically 2500 ppm glycerol monostearate, were assembled with a needle tip luer fitting. Assembly occurred at controlled axial forces of 3, 6, 8, 10 and 12 lb. (Although a resin having 2500 ppm was used for these samples, the same phenomenon has been observed in syringes made from resins with lower amount of GMS, for example, 1200 ppm and 600 ppm.) The hub pull force required to disassemble the syringe from the luer fitting was measured.

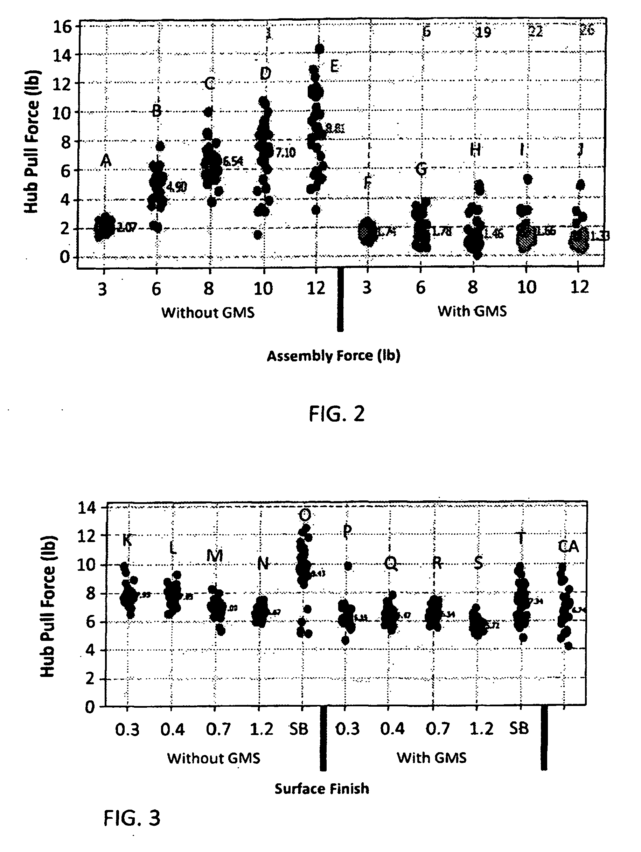

FIG. 2 shows a graph of the pull force required to disassemble a connected hub for Examples A-J under wet conditions. There were between 25 and 30 measurements taken at each data point. The data shows that the pull forces increased with increasing assembly force for materials without GMS. On the other hand, the pull forces for syringes with GMS remained fairly constant, with increasing spring back, regardless of the assembly force. Spring back is a phenomenon where the assembly force, on a luer slip syringe assembled to a female luer device, when removed, the syringes shows a sudden movement away from the female device. The number of samples exhibiting spring back is listed above each column of data points in FIG. 2.

### Examples K-O

Luer slip syringes made from a resin without glycerol monostearate or other lubricious agents were prepared with a variety of surface finishes. The surface finishes were prepared by Electro-Discharge Machining (EDM) or using a sandblasted tip insert. The average roughness of the EDM finished surfaces were 0.3, 0.4, 0.7 and 1.2 *µ*m. The syringes were assembled with a luer hub using an assembly axial force of 10 lbs. The force required to disassemble the syringe from the hub was measured.

### Examples P-T

Luer slip syringes made from a resin containing a lubricous agent, specifically glycerol monostearate, were prepared with a variety of surface finishes. The surface finishes were prepared by Electro-Discharge Machining (EDM) or a sandblasted insert. The average roughness of the EDM finished surfaces were 0.3, 0.4, 0.7 and 1.2 *µ*m. The syringes were assembled with a luer hub using an assembly axial force of 10 lbs. The force required to disassemble the syringe from the hub was measured.

### Comparative Example CA

For comparison purposes only, syringes from Terumo^{®} Medical Corporation were assembled with a luer hub using an assembly force of 10 lbs. The force required to disassemble the syringe from the hub was measured.

FIG. 3 shows a graph of the hub axial pull force (lbs) required to disassemble the syringes of Examples K-T and Comparative Example CA under wet conditions. The average surface roughness for the EDM prepared samples is listed below the chart. The surface roughness for the samples containing the sandblasted insert is not listed. FIG. 3 shows that the syringes which have glycerol monostearate and a surface finish (Examples P-T) show equivalent pull forces to the modified surface finished syringes that do not have GMS. The data also shows that after modifying the surface with EDM or adding a sandblasted tip insert, the pull forces are equivalent to the pull forced exhibited by a Terumo^{®} syringe.

### Examples U-Z

Luer slip syringes made from a resin containing a lubricious agent, specifically glycerol monostearate, were prepared with the surface finishes shown in Table 1. The syringes were assembled with a luer hub at assembly axial forces of 8, 10 and 12 lbs. The force required to disassemble the syringe from the hub was measured.

**Table 1.**

| *Sample* | *Surface Finish* |
|---|---|
| U | Unmodified |
| V | Sandblasted tip insert |
| W | EDM, Average Roughness = 0.3 *µ*m |
| X | EDM, Average Roughness = 0.4 *µ*m |
| Y | EDM, Average Roughness = 0.7 *µ*m |
| Z | EDM, Average Roughness = 1.2 *µ*m |

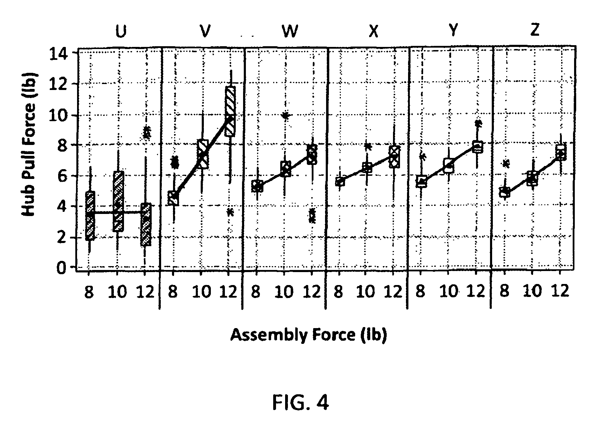

FIG. 4. Shows a graph of the hub pull forces for Samples U-Z under wet conditions. The data shows that the syringes with GMS and a surface finish (Samples V-Z) have a pull of force which increases with the assembly force, like the samples without a lubricious agent. (See Samples A-E in FIG. 2.) Whereas the group of samples that have GMS but no surface finish (Sample U) have relatively equivalent pull off forces regardless of the assembly force, as was previously shown in Samples F-J. (See FIG. 2.)

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

Although the invention herein has been described with reference to particular embodiments, it is to be understood that these embodiments are merely illustrative of the principles and applications of the present invention. It will be apparent to those skilled in the art that various modifications and variations can be made to the method and apparatus of the present invention without departing from the scope of the invention.

## Claims

1. A medical device (10) comprising a male luer slip fitting (12) connectable to a female luer portion (14) of a second medical device (16) by application of an engagement force to provide a contact surface between the male luer slip fitting (12) and female luer portion (14) and separable by application of a disengagement force that does not require substantial twisting motion, the male luer slip fitting (12) comprising a material incorporating a lubricious agent such that the disengagement force of a dry connection between the male luer slip fitting (12) and female luer portion (14) is less than the engagement force when the contact surface does not include a roughened surface, and the male luer slip fitting (12) has contact surface that is roughened such that the disengagement force required to separate the male luer slip fitting (12) from the female luer portion (14) of the second medical device (16) when the contact surface is wet is greater than the force required to disengage the male luer slip fitting (12) from the female luer portion (14) of the second medical device (16) under the same conditions when the contact surface of the male luer fitting (12) is not roughened, **characterized in that** the material of the male luer slip fitting (12) inclues an additive that enhances the lubricious property of the material, and the external surface of the male luer fitting (12) is roughened to between 0.3 µm to 1.2 µm.

2. The medical device (10) of claim 1, wherein the force required to disengage the male luer fitting (12) from the female luer portion (14) increases with increasing engagement force.

3. The medical device (10) of claim 1, wherein the force required to disengage the male luer fitting (12) from the female luer portion (14) is less than 80% of the engagement force.

4. The medical device (10) of claim 1, wherein the surface of the male luer (12) is roughened using a mold to form the tip that has been roughed using a technique selected from Electrodischarge Machining (EDM), vapor honing, cross hatching, polishing and combinations thereof.

5. The medical device (10) of claim 1, wherein the external surface of the male luer fitting (12) is roughened with a random pattern finish.

6. The medical device (10) of claim 1, wherein the additive is glycerol monostearate.

7. The medical device (10) of claim 1, wherein the additive is a slip agent.

8. The medical device (10) of claim 7, wherein the slip agent is a fatty acid amide.

9. A method of making a medical device (10) comprising:
manufacturing a male luer fitting (12) with a conical tip having an outside surface that mates with a female luer fitting (14) of a second medical device (16), male luer fitting manufactured from a material that includes an additive that enhances the lubricious properties of the material, such that the force required to disassemble an non-roughened male conical tip from a female luer fitting (14) is less than the force required to assemble the non-roughened male luer fitting (12) from the female luer fitting (14); and
roughening the outside surface of the male conical tip to a roughness of between 0.3 µm and 1.2 µm such that upon assembly of the male luer fitting (12) with the female luer fitting (14) with liquid present between the male conical tip and female luer fitting (14), the removal force is less than about the assembly force.

10. The method of claim 9, wherein the outside surface of the male luer fitting (12) is roughened with a random pattern finish.

11. The method of claim 9, wherein the surface of the male conical tip is roughened using a mold to form the tip that has been roughed using a technique selected from Electrodischarge Machining (EDM), vapor honing, cross hatching, polishing, sand blasting, bead blasting and combinations thereof.

12. The method of claim 9, wherein the additive is glycerol monostearate.

13. The method of claim 9, wherein the additive is a slip agent.

14. The method of claim 13, wherein the slip agent is a fatty acid amide.

## Patentansprüche

1. Medizinische Vorrichtung (10), die einen männlichen Luer-Slip-Anschluss (12) umfasst, der durch Anwendung einer Steckkraft mit einem weiblichen Luer-Teil (14) einer zweiten medizinischen Vorrichtung (16) verbunden werden kann, wobei eine Kontaktfläche zwischen dem männlichen Luer-Slip-Anschluss (12) und dem weiblichen Luer-Teil (14) entsteht, und durch Anwendung einer Lösekraft, die keine wesentliche Drehbewegung erfordert, lösbar ist, wobei der männliche Luer-Slip-Anschluss (12) ein Material, in das ein die Gleitfähigkeit erhöhender Stoff eingearbeitet ist, umfasst, so dass die Lösekraft einer trockenen Verbindung zwischen dem männlichen Luer-Slip-Anschluss (12) und dem weiblichen Luer-Teil (14) kleiner ist als die Lösekraft, wenn die Kontaktfläche keine aufgeraute Oberfläche umfasst, und der männliche Luer-Slip-Anschluss (12) eine Kontaktfläche aufweist, die so aufgeraut ist, dass, wenn die Kontaktfläche feucht ist, die Lösekraft, die erforderlich ist, um den männlichen Luer-Slip-Anschluss (12) vom weiblichen Luer-Teil (14) der zweiten medizinischen Vorrichtung (16) zu trennen, größer ist als die Kraft, die erforderlich ist, um den männlichen Luer-Slip-Anschluss (12) unter denselben Bedingungen, wenn die Kontaktfläche des männlichen Luer-Anschlusses (12) nicht aufgeraut ist, aus dem weiblichen Luer-Teil (14) der zweiten medizinischen Vorrichtung (16) zu lösen, **dadurch gekennzeichnet, dass** das Material des männlichen Luer-Slip-Anschlusses (12) ein Additiv umfasst, das die Gleiteigenschaften des Materials erhöht, und die äußere Oberfläche des männlichen Luer-Anschlusses (12) auf 0,3 µm bis 1,2 µm aufgeraut ist.

2. Medizinische Vorrichtung (10) gemäß Anspruch 1, wobei die Kraft, die erforderlich ist, um den männlichen Luer-Anschluss (12) vom weiblichen Luer-Teil (14) zu trennen, mit zunehmender Steckkraft zunimmt.

3. Medizinische Vorrichtung (10) gemäß Anspruch 1, wobei die Kraft, die erforderlich ist, um den männlichen Luer-Anschluss (12) vom weiblichen Luer-Teil (14) zu trennen, weniger als 80% der Steckkraft beträgt.

4. Medizinische Vorrichtung (10) gemäß Anspruch 1, wobei die Oberfläche des männlichen Luer-Anschlusses (12) unter Verwendung einer zur Bildung der Spitze dienenden Form aufgeraut wird, die mit einer Technik aufgeraut wurde, welche aus Funkenerodieren (EDM), Feuchtstrahlen, Kreuzschliff, Polieren und Kombinationen davon ausgewählt ist.

5. Medizinische Vorrichtung (10) gemäß Anspruch 1, wobei die äußere Oberfläche des männlichen Luer-Anschlusses (12) mit einem Zufallsmuster aufgeraut ist.

6. Medizinische Vorrichtung (10) gemäß Anspruch 1, wobei es sich bei dem Additiv um Glycerinmonostearat handelt.

7. Medizinische Vorrichtung (10) gemäß Anspruch 1, wobei das Additiv ein Gleitmittel ist.

8. Medizinische Vorrichtung (10) gemäß Anspruch 7, wobei das Gleitmittel ein Fettsäureamid ist.

9. Verfahren zur Herstellung einer medizinischen Vorrichtung (10), umfassend:
Herstellen eines männlichen Luer-Anschlusses (12) mit einer konischen Spitze, der eine äußere Oberfläche aufweist, die zu einem weiblichen Luer-Anschluss (14) einer zweiten medizinischen Vorrichtung (16) passt, wobei der männliche Luer-Anschluss aus einem Material hergestellt wird, das ein Additiv umfasst, welches die Gleiteigenschaften des Materials erhöht, so dass die Kraft, die erforderlich ist, um eine nicht aufgeraute männliche konische Spitze aus einem weiblichen Luer-Anschluss (14) zu lösen, kleiner ist als die Kraft, die erforderlich ist, um den nicht aufgerauten männlichen Luer-Anschluss (12) aus dem weiblichen Luer-Anschluss (14) zu montieren; und
Aufrauen der äußeren Oberfläche der männlichen konischen Spitze bis zu einer Rauigkeit zwischen 0,3 µm und 1,2 µm, so dass beim Montieren des männlichen Luer-Anschlusses (12) mit dem weiblichen Luer-Anschluss (14), wobei Flüssigkeit zwischen der männlichen konischen Spitze und dem weiblichen Luer-Anschluss (14) vorhanden ist, die Trennkraft kleiner ist als die ungefähre Zusammensetzkraft.

10. Verfahren gemäß Anspruch 9, wobei die äußere Oberfläche des männlichen Luer-Anschlusses (12) mit einem Zufallsmuster aufgeraut wird.

11. Verfahren gemäß Anspruch 9, wobei die Oberfläche der männlichen konischen Spitze unter Verwendung einer zur Bildung der Spitze dienenden Form aufgeraut wird, die mit einer Technik aufgeraut wurde, welche aus Funkenerodieren (EDM), Feuchtstrahlen, Kreuzschliff, Polieren und Kombinationen davon ausgewählt ist.

12. Verfahren gemäß Anspruch 9, wobei es sich bei dem Additiv um Glycerinmonostearat handelt.

13. Verfahren gemäß Anspruch 9, wobei das Additiv ein Gleitmittel ist.

14. Verfahren gemäß Anspruch 13, wobei das Gleitmittel ein Fettsäureamid ist.

## Revendications

1. Dispositif médical (10) comprenant un raccord à glissement Luer mâle (12) pouvant être raccordé à une partie Luer femelle (14) d'un second dispositif médical (16) par application d'une force de mise en prise pour fournir une surface de contact entre le raccord à glissement Luer mâle (12) et la partie Luer femelle (14) et pouvant être séparé par application d'une force de séparation ne nécessitant pas de mouvement de torsion sensible, le raccord à glissement Luer mâle (12) comprenant un matériau contenant un agent lubrifiant de telle sorte que la force de séparation d'un raccordement à sec entre le raccord à glissement Luer mâle (12) et la partie Luer femelle (14) est inférieure à la force de mise en prise lorsque la surface de contact ne comprend pas une surface rugueuse, et le raccord à glissement Luer mâle (12) a une surface de contact qui est rugueuse de telle sorte que la force de séparation nécessaire pour séparer le raccord à glissement Luer mâle (12) de la partie Luer femelle (14) du second dispositif médical (16) lorsque la surface de contact est humide est supérieure à la force nécessaire pour séparer le raccord à glissement Luer mâle (12) de la partie Luer femelle (14) du second dispositif médical (16) dans les mêmes conditions lorsque la surface de contact du raccord à glissement Luer mâle (12) n'est pas rugueuse, **caractérisé en ce que** le matériau du raccord à glissement Luer mâle (12) comprend un additif qui améliore la propriété lubrifiante du matériau et la surface externe du raccord à glissement Luer mâle (12) a une rugosité entre 0,3 µm et 1,2 µm.

2. Dispositif médical (10) selon la revendication 1, dans lequel la force nécessaire pour séparer le raccord Luer mâle (12) de la partie Luer femelle (14) augmente lorsque la force de mise en prise augmente.

3. Dispositif médical (10) selon la revendication 1, dans lequel la force nécessaire pour séparer le raccord Luer mâle (12) de la partie Luer femelle (14) est inférieure à 80 % de la force de mise en prise.

4. Dispositif médical (10) selon la revendication 1, dans lequel la surface du Luer mâle (12) est rugosifiée à l'aide d'un moule pour former la pointe qui a été rugosifiée à l'aide d'une technique choisie parmi l'usinage à électrodécharge (EDM), le rodage à la vapeur, le quadrillage, le polissage et des combinaisons de ceux-ci.

5. Dispositif médical (10) selon la revendication 1, dans lequel la surface externe du raccord Luer mâle (12) est rugosifiée avec un fini à motifs aléatoires.

6. Dispositif médical (10) selon la revendication 1, dans lequel l'additif est du monostéarate de glycérol.

7. Dispositif médical (10) selon la revendication 1, dans lequel l'additif est un agent glissant.

8. Dispositif médical (10) selon la revendication 7, dans lequel l'agent glissant est un amide d'acide gras.

9. Procédé de fabrication d'un dispositif médical (10) comprenant :
la fabrication d'un raccord Luer mâle (12) à pointe conique ayant une surface extérieure qui s'adapte à un raccord Luer femelle (14) d'un second dispositif médical (16), raccord Luer mâle fabriqué à partir d'un matériau qui comprend un additif qui améliore les propriétés lubrifiantes du matériau, de telle sorte que la force nécessaire pour désassembler une pointe conique mâle non rugueuse d'un raccord Luer femelle (14) est inférieure à la force nécessaire pour assembler le raccord Luer mâle non rugueux (12) du raccord Luer femelle (14) ; et
la rugosification de la surface extérieure de la pointe conique mâle à une rugosité comprise entre 0,3 µm et 1,2 µm de telle sorte que lors de l'assemblage du raccord Luer mâle (12) avec le raccord Luer femelle (14) avec du liquide présent entre la pointe conique mâle et le raccord Luer femelle (14), la force de retrait est environ inférieure à la force d'assemblage.

10. Procédé selon la revendication 9, dans lequel la surface extérieure du raccord Luer mâle (12) est rugosifiée avec un fini à motifs aléatoires.

11. Procédé selon la revendication 9, dans lequel la surface de la pointe conique mâle est rugosifiée en utilisant un moule pour former la pointe qui a été rugosifiée à l'aide d'une technique choisie parmi l'usinage à électrodécharge (EDM), le rodage à la vapeur, le quadrillage, le polissage, le sablage, la projection de billes et des combinaisons de ceux-ci.

12. Procédé selon la revendication 9, dans lequel l'additif est du monostéarate de glycérol.

13. Procédé selon la revendication 9, dans lequel l'additif est un agent glissant.

14. Procédé selon la revendication 13, dans lequel l'agent glissant est un amide d'acide gras.
